# EUROPÄISCHE PATENTANMELDUNG

(11) **EP 1 338 245 A2**
(43) Veröffentlichungstag der Anmeldung: **27.08.2003**
(21) Anmeldenummer: 03002964.9
(22) Anmeldetag: 11.02.2003
(51) Int. Cl.: A61B 5/05, G01G 19/414, G01G 19/50

(54) **Darstellung des Mess-Ergebnisses eines Körperfettmessgeräts**

(30) Priorität: 13.02.2002 DE 10205823
(71) Anmelder: Soehnle-Waagen GmbH & Co. KG, 71540 Murrhardt (DE)
(72) Erfinder: Nahm, Werner, Dr., 74426 Bühlerzell (DE)

(57) **Zusammenfassung**

Die Erfindung betrifft ein Verfahren zur Darstellung des Meßergebnisses eines Körperanalysegerätes, insbesondere einer Körperanalysewaage. Zur Bewertung des Meßergebnisses wird vorgeschlagen, daß die Kombination aus zumindest einem physiologischen und zumindest einem anthropometrischen Parameter über einen Transponder in einer in zumindest drei Gruppen unterteilten, interpretierenden Grafikanzeige dargestellt ist.

## Beschreibung

Die Erfindung betrifft ein Verfahren zur Darstellung des Mess-Ergebnisses eines Körperanalysegerätes, insbesondere einer Körperanalysewaage.

Bisher wird auf Körperanalysewaagen, insbesondere auf Körperfettmesswaagen und anderen Geräte zur Analyse der Körperzusammensetzung neben der Anzeige des Körpergewichtes in kg ein weiterer Parameter, in der Regel der Körperfettanteil in Prozent angezeigt. Die Anzeige der beiden numerischen Werte erfolgt entweder simultan oder abwechselnd. In einigen Fällen wird zusätzlich, zum Beispiel in Form einer Balkenanzeige, eine Interpretationshilfe zur Einschätzung des Körperfettanteiles angeboten. Diese Interpretationshilfe ist bei einem marktgängigen Gerät als unterbrochener, ansteigender Balken ausgeführt, neben dem die Anzeige in Prozent erfolgt. Bei einem anderen marktgängigen Gerät ist eine Anzeige mit den Hinweisen niedrig-normal-hoch dargestellt.

Diese Art der Anzeigen ist aber insofern problematisch, als die dieses Gerät benutzende Person, um festzustellen, ob das ermittelte Körpergewicht für sie niedrig, normal, oder zu hoch ist, nach Empfehlung der Welt-Gesundheitsorganisation, den Body-Maß-lndex (BMI) berechnen muss, wozu in einer Tabelle zunächst der für das jeweilige Geschlecht und Alter empfohlene Normalbereich abgelesen werden muss. Noch unübersichtlicher wird der Vorgang bei Feststellen des für die jeweilige Person maßgeblichen Körperfettanteils. Auch hier muss umständlich in Tabellen nachgeforscht werden.

Noch weitaus schwieriger ist es für den Benutzer einer Körperfettmesswaage, die beiden Parameter Gewicht- und Körperfettgehalt im Zusammenhang richtig zu interpretieren. Dies leicht nachvollziehbar, weil der normale Benutzer derartiger Geräte in aller Regel ein medizinischer Leihe ist. Dabei können leicht Missverständnisse entstehen und unter Umständen die falschen Konsequenzen aus der Kombination der beiden Parameter gezogen werden.

Zum Beispiel könnte ein Benutzer, dessen Körpergewicht nach dem Body-Maß-lndex als zu niedrig eingestuft ist, dessen prozentualer Körperfettanteil jedoch laut Tabelle zu hoch ist, fälschlicherweise annehmen, sein Körper sei zu fett. Die Folge könnte sein, dass der Benutzer weiter versucht, abzunehmen, und dies zu ernsthaften gesundheitlichen Schäden führen kann. Tatsächlich hat dieser Benutzer aber nicht zu viel Fett, sondern entsprechend seinem Gewicht zu wenig Muskelmasse. Es wäre deshalb fatal, wenn dieser Nutzer aufgrund einer Fehlinterpretation eine Diät durchführen würde, die sein ohnehin schon zu niedriges Körpergewicht noch weiter reduzieren würde. Statt dessen müsste er seine Messergebnisse zum Anlass nehmen, gezielt ein Muskelaufbautraining durchzuführen und damit sein Gewicht in den Normalbereich zu bringen. Durch dieses Muskelaufbautraining wird sein Körperfett natürlich nicht erhöht, so dass sein prozentualer Körperfettgehalt automatisch sinken würde und in einen normal zu bezeichnenden Bereich käme.

Aufgabe der Erfindung ist es, dem Benutzer obiger Geräte eine Hilfe an Hand zu geben, mit der er ohne medizinische Vorkenntnisse und ohne Nachsehen in Tabellen und / oder Broschüren leicht das Messergebnis in seiner weiteren Bedeutung für seine Gesundheit auswerten und bewerten kann.

Diese Aufgabe wird mit dem kennzeichnenden Merkmal des Anspruchs 1 gelöst.

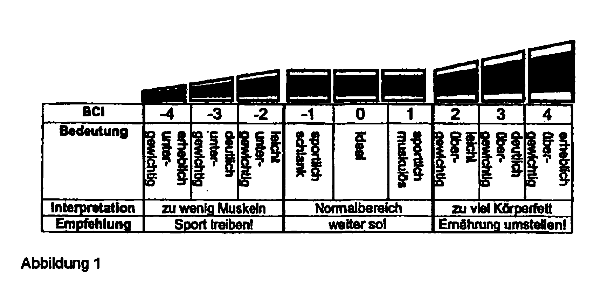

Durch die Eingabe von Alter, Geschlecht und Körpergröße kann auf der Basis der veröffentlichten Empfehlungen der Welt-Gesundheitsorganisation und den Empfehlungen von Ernährungswissenschaftlern mittels spezieller Formeln der individuelle Idealbereich für beide Parameter im Zusammenhang berechnet werden. Weiter kann die Abweichung vom Idealbereich für jeden Benutzer individuell ermittelt werden. Vorzugsweise werden alle Informationen durch spezielle Formeln zu einem Index zusammengefasst. In Abbildung 1 wird dieser Index als Body Composition Index bezeichnet (BCI). Der BCI nimmt für Parameter, die im persönlichen Idealbereich oder im risikofreien (gesunden) Bereich liegen den Wert Null an. Abweichungen vom individuellen Idealzustand ergeben einen BCI ungleich Null.

Für diese Abweichungen gibt es prinzipiell vier unterschiedliche Bereiche, wie in Tabelle 1 dargestellt.

Ein erster Bereich sind die Personen mit Untergewicht aber verbunden mit zu hohem Körperfettanteil. Man könnte sie als Diätgeschädigte bezeichnen.

Ein zweiter Bereich hat ebenfalls niedriges Körpergewicht aber dieses niedere Körpergewicht ist mit einem hohen Muskelanteil verbunden. Diesen Bereich könnte man als Marathonläufer bezeichnen.

Der nächste Bereich hat ein hohes Körpergewicht, dieses hohe Körpergewicht ist aber mit einem hohen Muskelanteil verbunden. Diesem Bereich sind zum Beispiel die sogenannten Bodybuilder zuzuordnen.

Schließlich bleibt als vierter Bereich die Personengruppe, die ein zu hohes Körpergewicht, also Übergewicht mit zu hohem Körperfettanteil, ausweist. Dieser Zustand wird medizinisch als Adipositas bezeichnet.

Daraus folgert, dass diese Erkenntnis und eine entsprechend verständliche Darstellung für den Benutzer der genannten Geräte besonders wichtig ist

Dabei liegen die Bereiche Untergewicht und Übergewicht jeweils verbunden mit zu hohem Körperfettanteil außerhalb des Idealbereiches und bilden die Risikogruppen.

In den beiden anderen Bereichen hoher Muskelanteil mit zu niedrigem oder zu hohem Körpergewicht finden sich im wesentlichen die Leistungssportler wieder. Diese Bereiche werden erfindungsgemäß als normal angezeigt und daher nicht weiter untergliedert.

Wichtig sind die beiden Bereiche Untergewicht und Übergewicht mit jeweils zu hohem Fettanteil, die jeweils mit Balken unterschiedlicher Stärke untergliedert sind.

Erfindungsgemäß erscheint deshalb im Display der Fettmesswaage nach Beendigung der Messung von Körpergewicht und Körperfettanteil zeitgleich zur Anzeige der numerischen Messwerte eine Balkengraphik in Form einer mehrteiligen Rampe, in dem in der Zeichnung dargestellten Beispiel bestehend aus neun Balkenelementen. Während die Konturen aller Balkenelemente dauemd sichtbar sind, wird nur jeweils ein Element gefüllt, d.h. aktiv angezeigt. Um den Benutzer nicht zu überfordern, sind zweckmäßigerweise drei Interpretationsbereiche ausgewiesen. Nämlich zu wenig Muskeln mit der Empfehlung zum Beispiel Sport zu treiben, ein Normalbereich mit der Empfehlung, weiter so, und schließlich der Bereich mit zu viel Körperfett mit der Empfehlung, Ernährung umstellen. Natürlich kann noch jeweils die Bedeutung, wie in der Figur ausgeführt, mit aufgenommen werden.

Schließlich ist es auch denkbar, die Unterteilung noch feiner vorzunehmen, wobei dies auch alternativ in einer entsprechenden Bedienungsanleitung erfolgen kann, so dass das Gerät selbst nicht überladen wird und nur der noch Gesundheitsbewusstere weitere Informationen erhalten kann.

In dem Ausführungsbeispiel gemäß Fig. 1 wird stilisiert ein Display dargestellt, das mit einem dauernd sichtbaren Balkenelement arbeitet, wobei die drei Interpretationsbereiche nochmals jeweils in drei Balkenbereiche unterteilt sind. Dabei sind der linke und der rechte Balkenbereich ansteigend ausgeführt, während der Normalbereich gradlinig verläuft. Zusätzlich ist der Body-Composition-Index (BCI), die Bedeutung der einzelnen Werte des Body-Composition-Index stichwortartig wiedergegeben. Wie bereits angedeutet, könnte natürlich diese Display-Balkengraphik mit weiteren Informationen versehen sein.

Bevorzugt könnte auch der untergewichtige Bereich mit einem sterilisierten Gewichtheber, der Normalbereich mit einer Waage und der übergewichtige Bereich mit Obst erläutert sein.

## Patentansprüche

1. Verfahren zur Darstellung des Mess-Ergebnisses eines Körperanalysegerätes, insbesondere einer Körperanalysewaage, **dadurch gekennzeichnet, dass** die Kombination aus zumindest einem physiologischen und zumindest einem anthropometrischem Parameter über einen Transponder in eine in zumindest drei Gruppen unterteilten, interpretierenden Graphikanzeige dargestellt ist.

2. Verfahren nach Anspruch 1, **dadurch gekennzeichnet, dass** die interpretierende Graphikanzeige durch zumindest jeweils eine Empfehlung erweitert ist.

3. Verfahren nach einem der Ansprüche 1 bis 2, **dadurch gekennzeichnet, dass** die erste Gruppe und dritte Gruppe der Graphikanzeige ansteigend, die mittlere Gruppe gleichmäßig verlaufend ausgeführt ist.

4. Verfahren nach Anspruch 1 bis 2, **dadurch gekennzeichnet, dass** die Gruppen mehrfach graduierend unterteilt sind.

5. Verfahren nach einem der Ansprüche 1 bis 4, **dadurch gekennzeichnet, dass** die Konturen der Graphikanzeige dauernd sichtbar sind und das jeweils das Messergebnis anzeigende Teilelemente einer Gruppe gefüllt (aktiv) ist.

6. Verfahren zur Darstellung des -Mess-Ergebnisses eines Körperanalysegerätes, insbesondere einer Körperanalysewaage, **dadurch gekennzeichnet, dass** die Kombination aus zumindest einem physiologischen und zumindest einem anthropometrischem Parameter über einen Transponder in Form eines numerischen Index dargestellt wird.

7. Verfahren nach Anspruch 6, **dadurch gekennzeichnet, dass** der Index für den Zielzustand oder Idealzustand den Wert Null einnimmt.

8. Verfahren nach den Ansprüchen 6 und 7, **dadurch gekennzeichnet, dass** die Abweichung vom Idealzustand durch einen numerischen Wert ungleich Null angezeigt wird.

9. Verfahren nach den Ansprüchen 6,7 und 8, **dadurch gekennzeichnet, dass** die Stärke der Abweichung, insbesondere die Höhe eines gesundheitlichen Risikos durch den Betrag der Abweichung angezeigt wird.
